# EUROPEAN PATENT SPECIFICATION

(11) **EP 1 260 187 B1**
(45) Date of publication and mention of the grant of the patent: **18.04.2007**
(21) Application number: 00923032.7
(22) Date of filing: 03.02.2000
(51) Int. Cl.: A61B 17/66, A61B 17/88, A61B 17/64, A61B 17/70

(54) **DEVICE FOR EXTERNAL TRANSPEDICULAR VERTEBRAL COLUMN FIXATION**
VORRICHTUNG ZUR EXTERNEN TRANSPEDIKULAREN WIRBELSÄULENFIXIERUNG
DISPOSITIF UTILISE POUR FIXATION EXTERNE, TRANSPEDICULAIRE, DE COLONNE VERTEBRALE

(43) Date of publication of application: 27.11.2002
(73) Proprietor: Fed. State Institution of Science Russian Ilizarov Scient. Ctr. Restorative Traumatology & Orthopaed. Federal Agency of Health & Social Development, Kurgan 640005 (RU)
(72) Inventor: SHEVTSOV, Vladimir Ivanovich, Kurgan, 640020 (RU); KHUDYAEV, Alexandr Timofeevich, Kurgan, 640023 (RU); KOVALENKO, Pavel Ivanovich, Kurgan, 640020 (RU)
(74) Representative: Hano, Christian
(86) International application number: PCT/RU2000/000036
(87) International publication number: WO 2001/056488

(56) References cited:
- WO-A1-95/05786
- DE-A- 2 834 891
- RU-C- 2 066 140
- RU-C- 2 128 021
- RU-C1- 2 066 140
- SU-A- 839 515
- SU-A1- 1 409 249
- SU-A1- 1 644 928
- SU-A1- 1 648 418
- SU-A1- 1 768 147

## Description

### Field of the Invention

The invention relates to the field of medicine, specifically to that of orthopaedics and traumatology and exactly to spinal surgery, and is used for treatment of diseases and injuries of the spine in specialized departments of vertebrology and neurosurgery.

### Description of the Prior Art

An implanted device for spinal fixation is known, comprising a screw for spongy bone, bearing an improved saddle-shaped configuration, fixed in a place with a nut, and comprising a simple and effective locking mechanism, having some pins, each of them contacts with an upper half-saddle and a nut to prevent rotation of the latter with respect to the rest of the configuration. The implanted system also comprises rods, rigidly catched by the saddle-shaped configuration and sublaminar wire, where the rod/wire interface is protected with a wire tread (U.S. Patent No. 5,030,220. IPC⁵ A 61 F 5/04, A 61 F 5/00).

A rod screw is known as well as a device for correction and fixation with that kind of a screw, in which the rod screw has a threaded part and a spherical segment or a head, on the latter a packing is set with a connecting element to set a rod, and the connecting element and the packing are connected with each other in such a way, that the distance between its end and the connecting element can be changed (International Application N WO 91/06254. IPC⁵ A 61 B 17/60).

A device for spinal treatment is known, comprising junctions of vertebral fixation with curved needles, connected by straight screws with each other, and two units, which are connected with central plates, on which straight needles are fastened with the help of plates held down and screws (Author Certificate /A.c./ SU 1537237, A 2, USSR. IpC⁵ A 61 B 17/60). In the known device the central plates are made as a semicircle, supplied with holes and cut-outs. Moreover, vertebral fixation is performed, using curved needles, inserted into spinous process, and straight needles, inserted into vertebral body.

A technique of modelling of closed insertion of fixators in vertebral body is known, comprising its stabilization by insertion of wire-markers in its spinous process in the middle sagittal plane, after that, displacing from it at first in the cranial and then in the lateral direction the fixators are inserted into safe zones from backward forward at an angle to the sagittal plane, and their direction is marked on a template, which is then used during insertion of the fixators (Patent, RU 2093901, C1, RF).

A technique for insertion of a threaded rod in vertebral body is known as well as a device for this implementation (Patent RU 2051633, C1, RF). The essence of the invention is in preinsertion of guiding axial wire, a guide with a central longitudinal hole, a protective tube, with the help of which protection of soft tissues is made, direction and depth of threaded rod implantation are controlled, for this a device for osteosynthesis is used, provided with a rotation mechanism as a case, including a ratchet with a lock, a quadrangular jack with a ball fixator on one end and a handle with circular recesses, set on the opposite end perpendicular to the case.

A compression-distraction device is known, including rings and arches, wire fixators, tie screws, spherical hinges, fixing elements, in which each pair of the tie screws is connected by the spherical hinge, and the rings and arches are set at a regulated distance from the plane of the hinge arrangement (A.c. SU 374076, USSR).

A device for fixation and correction of the spine is known, comprising threaded rods and a base, provided with lever ties (Patent RU 2019148, C1, RF). There are longitudinal threaded bars in the known device, each of them is made being consisted of three parts, connected with each other by hinges and provided with rod holders as a cylinder with a fixing element and a slot, thereby the parts provide multiplanar transport of the threaded rods.

A device for treatment of scoliosis is known, comprising staples with holes and a slot, attached to them support elements and fixation elements, in which one of the staple ends is deviated along the plane to its convex side, a fixing plate with L-reversed section is arranged on the staple with the possibility of its transport along the slot and fixation; a slider is on one of its wall, it is sunk into the staple slot and becomes an axle, in the top of which, in its turn, a threaded pole is made, and a slot, parallel with the staple plane, is made in the other wall of the fixing plate, a tractor is set by hinges on the axle of the fixing plate, its threaded end is attached to the deviated end of the staple through a face support, bone rods and clips are in the slots of the fixing plates with the possibility of transport and fixation, the latters consist of two arched plates with tenons, connected by a tie bolt at their base, and the staples are connected with each other with the possibility of their interlocation change and fixation by use of hinge bars (Patent RU 2086202, C 1, RF).

A device and a technique for spinal extrafocal osteosynthesis are known, the device comprises threaded rods, support elements, screw tractions and fixing elements, the support elements in the device are made as arched plates with curvature radius 140 ± 10 mm and longitudinal slots, in which the threaded rods are fastened perpendicular to the plane of the support plate, the threaded rods are to be inserted in vertebral bodies, free movements are made within 30°- range with respect to the support plate at the expense of compensatory washers, the support elements have free movements within 60°- range and are connected with each other by posts with the compensatory washers and the longitudinal screw tractions in two parallel planes. In the technique of spine extrafocal osteosynthesis by transpedicular insertion of the threaded rods in bodies of some adjacent intact vertebrae with attachment to the external support transpedicular fixation is supplemented by fixation of spinous process, in an involved vertebra the rods are inserted directly in the body, omitting the arch, and two adjacent vertebrae are fixed by a single support plate, for example, the involved vertebra and the one below, thereby creating repositional moment of forces at the level of the most involved spinal motor segment (Patent RU 2115381, CI, RF).

ESSF device is known, which has transverse beams, made integral and provided with apertures to set ball hinges with lock screws (Weber B.G., Magenl F. The external fixator. AO/ASIF - Thread Rod System Spine - Fixator. Berlin: Springer-Verlag, 1985. - P. 298-300). The transverse beams are connected with each other by threaded rods and the ball hinges. All the threaded rods have antirotational thread. In the device Schanz screws are used, which are connected by the transverse beams, using screw clamps. Slots, made in the transverse beams, give the possibility to change transverse distance of the Schanz screws.

However, the design of the known device doesn't allow to correct spinal deformities and exactly scoliosis of III-IV degree, because setting of the ball hinges in the transverse beam apertures limits its use versatility due to absence of the possibility of setting ball hinges, oriented to the deformity apex.

A device is known for infusion of medicamentous solutions into bone and soft tissues, comprising a case with a tubular needle, a union for solution feeding in and a mandrin with a cone plug and a handle, set with the possibility of rotation around the axis, a longitudinal groove and as a perpendicular to it narrowing capillary gap are made on its mandrin cone plug, the mandrin with the needle form a throttling valve, the flow section of which is the same as the cross-section of the longitudinal groove (A. c. SU 858839, USSR. IPC³ A 61 M 1/00).

A device is known for intraosseous injections, comprising a handle with a longitudinal canal and a slot, a separable needle, set on the handle, a mandrin with a slider, set with the possibility of transport in the handle and needle canals, and a fixator of the mandrin position, in which a handle is provided with two stops at its ends, the fixator is set on the stop of the handle working end and the shape of the handle working end conforms with the shape of the mandrin working end (A. c. SU 914063, USSR. IPC⁴ A 61 M 1/00).

However, the design of the known device is intended for independent use and it is not provided for its use in combination with the vertebral fixation element.

A clamp is known, comprising two handles, connected by hinges, with rings and a rack-and-pinion lock and working jaws, where the working jaws are made with cheek-pieces and slotted bottom, moreover, the closed slots form a round hole (A. c. SU 850064, USSR. IPC³ A 61 B 17/18).

However, design of the working jaws doesn't allow to use the known clamp for insertion of the fastening end of the vertebral fixation element through soft tissues to vertebral body.

A device for scoliosis treatment is known, comprising supports, set in parallel and interconnected, on which elements of vertebral fixation are fastened, in which each support is made as a plate, provided with connecting shanks, the latters are fastened in posts, set with the possibility of transport in slots of schwellers, moreover, each preceding support is connected with each following one with the possibility of transport (Certificate 1797 for utility model, RF. IPC⁶ A 61 B 17/60).

A correcting device for the spine is known, comprising a unit of vertebral fixation, which is made from a support as two bars, connected with each other by a central support element and at the ends, having lateral support elements. In the longitudinal slots of the bars clamps with vertebral fixators are set (Patent RU 2128021, C1 RF. IPC⁶ A 61 B 17/66, 17/70).

A repositioning apparatus for applying dosed traction to spine is known, comprising four fixation units forming two support units (Patent RU2066140, C1, RF). The preamble of claim 1 is based on this document.

However, the design of the known devices hasn't sufficient stiffness due to the fixation junction, having a large number of built-up elements.

### Summary of the Invention

It is an object of the present invention to work out design of a device for external transpedicular fixation of the spine for correction of its deformities and injuries, an instrument for insertion of the vertebral fixation element, and a technique of their use for treatment of deformities and injuries of the spine.

The object is achieved in accordance with the fact, that in the device for external transpedicular fixation of the spine there are not more than 9 fixation junctions, fastened by ties in threes as three support units, two of them are outer and one - intermediate, each of them is connected with each other with the possibility of spatial transport with the help of not more than 6 distraction rods, the ends of each out of three distraction rods are indirectly connected with one of the fixation junctions of the outer support units and, respectively, with the fixation junctions of the intermediate support unit, moreover, each of the fixation junctions is made as a figure plate, having a transverse axis of symmetry and provided with symmetric through longitudinal slots, located between a central bulge and ends, made as a prism, the central bulge and the ends as a prism are provided with connecting holes and fastening elements, moreover, to each of the fixation junctions not more than two elements for vertebral fixation are attached by clamps, the elements are made as half pins, each of them has a working end, made cone-shaped and provided with wood screw-shaped thread, limited by a stopper, and a fastening end, on which flattening is made, provided with a through hole, axis of the latter is perpendicular to longitudinal axis of the half pin.

Preferably each of the fixation junctions is made as a figure plate with its shape as an isosceles trapezoid and, respectively, transverse symmetry axis of the plate, provided with symmetric through connecting holes, axis of each of the latters is perpendicular to the transverse symmetry axis, the connecting holes are made in the middle and at the ends of each plate, moreover, each of the latters is provided with two pairs of through mounting slots, made at angle 35-40° on both sides from the transverse symmetry axis of the plate, in the slots, in one out of the pair, an element of vertebral fixation is fastened by one, made as a half pin, the outer support units are connected with the intermediate one by connecting hinge junctions, each of them is assembled from interconnected posts, and each of the latters is provided with distraction rods.

Such an implementation of the fixation junctions of the mentioned device allows to increase stiffness of fixation of intact vertebrae, using the elements of their fixation, fastening ends of which are oriented at angle 35-40° with respect to the spinal longitudinal axis, and to regulate arrangement of fixing site of the fastening ends of the fixation elements of vertebrae in view of applied distraction forces.

Simultaneously the object is achieved to infuse medicamentous solutions in bodies of vertebrae during their fixation in the process of treatment at the expense of the fact, that each half pin is provided with a longitudinal slot, made all over the half pin length, and in the slot a syringe needle is fixed, in which a cannula, provided with a plug, is set from the direction of the fastening end of the half pin.

To provide achievement of complete correction of spinal deformity both for trauma and for scoliosis of 3-4 degree preferably to make each of the connecting hinge junctions as a ball head having cylindrical bulges with a hole, the head is set on the side, truncated in the direction, opposed to the cylindrical bulge, and a hollow sphere, the inner surface of which is covered with antifriction coating, distraction rods are set in the holes of the cylindrical bulges, the truncated part of the hollow sphere is 1/3 of the hollow sphere and the angle of the truncated part in projection is 90°, moreover, the ball head is provided with hollows and the hollow sphere - with a hole, the axis of which is perpendicular to the axis of the holes of the cylindrical bulges, in which a lock is set.

Moreover, each of the connecting hinge junctions can be made as a distraction rod, provided with a ball head in one of the ends, the head is set in a sleeve, connected with a pivot, made on one of the ends of the second distraction rod.

In a variant of the invention implementation the device can be provided with a microschweller, on a plane of which a longitudinal slot is made and at least two mounting holes on each side from it, the microschweller is connected with the outer support units by two posts and threaded rods, and it is connected with the intermediate support unit by a lug with a threaded shank with the possibility of transport in the sagittal plane.

Implementation of the device with such a traction provides additional influence during correction of spinal lateral deformity and increases stiffness of the design.

To prevent turning-through of each fixation element of vertebra during its fastening on the fixation junction each of the clamps is preferably made as an angulate post, formed from the cylindrical threaded surface, connected at an angle with the prismatic head, and cylindrical turning-down is made on the inner surface in the direction of the rod, axis of the turn-down is perpendicular to the axis of the cylindrical threaded surface, on which conical and spherical washers can be set.

### Brief Description of the Drawings

The patented invention will be made clear by the following detailed description of each specific case of implementation with references to the accompanying drawings and diagrams.
Figure 1 is a configuration of the device for external transpedicular fixation of the spine according to the invention, general view;
Figure 2 is a fixation junction according to the present invention.
Figure 3 is a possible variant of the fixation junction implementation;
Figure 4 illustrates a possible variant of the configuration of the device for external transpedicular fixation of the spine;
Figure 5 shows an element for vertebral fixation, made as a half pin, according to the invention;
Figure 6 illustrates a possible variant of implementation of the vertebral fixation element;
Figure 7 is the same element for vertebral fixation as that in Fig. 6, top view;
Figure 8 shows a possible variant of implementation of a connecting ball hinge, according to the invention;
Figure 9 is a possible variant of implementation of the connecting ball hinge, according to the invention;
Figure 10 is an additional picture of the same connecting ball hinge;
Figure 11 is a possible variant of the configuration of the device for external transpedicular fixation of the spine, using a microschweller, according to the invention;
Figure 12 is an implementation of the microschweller, according to the invention, the same as in Fig. 10;
Figure 13 shows a clamp for fastening of the element for vertebral fixation to the fixation junction, according to the invention;
Figure 14 is a connecting element, made as a lug with a threaded shank;
Figure 15 is a surgical clamp for insertion of the element for vertebral fixation, according to the invention;
Figure 16 is a picture of jaws of the surgical clamp handles with a fixed flattened end of the vertebral fixation element;
Fig. 17 is a diagram of deformed spine, according to the invention;
Figure 18 is the same as in Fig. 17 after correction of the spinal deformity, using the device for external transpedicular fixation;
Figure 19 shows the fixation element of a vertebra, inserted in its body.

### Description of Preferred Implementation of the Invention

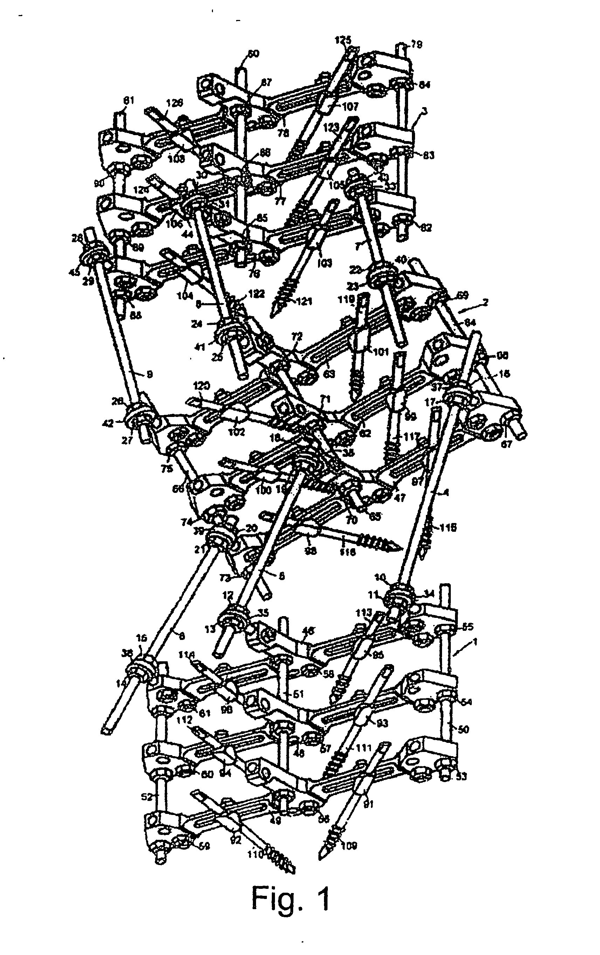

Let's refer now to Fig. 1 of the accompanying drawings, which illustrates the device for external transpedicular fixation of the spine as a whole.

The device comprises three support units 1, 2, 3, not more. In this configuration the support units 1 and 3 are outer, and the support unit 2 between them is intermediate. Support units 1, 2, 3 are connected with each other with the possibility of relative transport, using distraction threaded rods 4, 5, 6, 7, 8, 9, distraction nuts 10 and 11, 12 and 13, 14 and 15, 16 and 17, 18 and 19, 20 and 21, 22 and 23, 24 and 25, 26 and 27, 28 and 29, 30 and 31, 32 and 33, and, respectively, lugs 34, 35, 36, 37, 38, 39, 40, 41, 42, 43, 44, 45 with threaded shanks. Lugs 34, 35, 36 with the threaded shanks are set with the possibility of their turn in the holes of fixation junction 46 of outer support unit 1, and lugs 37, 38, 39 with their threaded shanks are set with the possibility of their turn in the holes of fixation junction 47 of intermediate support unit 2.

Support unit 1 is configured from three fixation junctions 46, 48, 49, which are connected with each other by threaded ties 50, 51, 52 and, respectively, by pairs of fastening nuts 53, 54, 55, located on threaded tie 50, by pairs of fastening nuts 56, 57, 58, located on threaded tie 51, and by pairs of fastening nuts 59, 60, 61, located on threaded tie 52.

Support unit 2 is configured from three fixation junctions 47, 62, 63, which are connected with each other by threaded ties 64, 65, 66 and, respectively, by pairs of fastening nuts 67, 68, 69, located on threaded tie 64, by pairs of fastening nuts 70, 71, 72, located on threaded tie 65, and by pairs of fastening nuts 73, 74, 75, located on threaded tie 66.

Lugs 34, 35, 36 and 37, 38, 39 are located on distraction threaded rods 4, 5, 6 and can be transported by the distraction nuts in pairs, namely: 10 and 11, 12 and 13, 14 and 15, 16 and 17, 18 and 19, 20 and 21.

Lugs 40, 41, 42 are located on distraction threaded rods 7, 8, 9 and can be transported by the distraction nuts in pairs, namely: 22 and 23, 24 and 25, 26 and 27. Moreover, lugs 43, 44, 45 are also located on distraction threaded rods 7, 8, 9 and can be transported by the distraction nuts in pairs, namely: 28 and 29, 30 and 31, 32 and 33. The threaded ends of lugs 43, 44, 45 are set with the possibility of transport in the holes of fixation junction 76 of outer support unit 3.

The latter is configured from three fixation junctions 76, 77, 78, which are connected with each other by threaded ties 79, 80, 81 and, respectively, by pairs of fastening nuts 82, 83, 84, located on threaded tie 79, by pairs of fastening nuts 85, 86, 87, located on threaded tie 80, and by pairs of fastening nuts 88, 89, 90, located on threaded tie 81.

The elements for vertebral fixation in twos, and namely: 109, 110, 111, 112, 113, 114, 115, 116, 117, 118, 119, 120, 121, 122, 123, 124, 125, 126, are fastened, respectively, on each fixation junction 46, 47, 48, 49, 62, 63, 76, 77, 78, using the clamps, and namely: 91, 92, 93, 94, 95, 96, 97, 98, 99, 100, 101, 102, 103, 104, 105, 106, 107, 108.

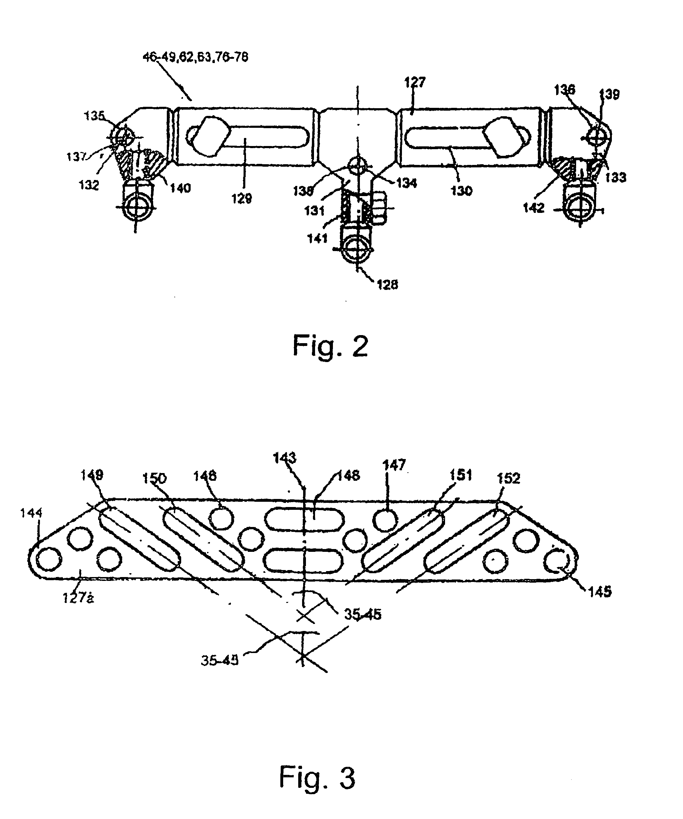

Each of fixation junctions 46, 47, 48, 49, 62, 63, 76, 77, 78 is made as symmetrical figure plate 127 with transverse axis of symmetry 128 (Fig. 2). In the plate symmetrical through longitudinal slots 129 and 130 are made in twos, they are located between central bulge 131 and ends 132 and 133 of figure plate 128. Connecting holes 134, 135, 136 in one are made in central bulge 131 and in each of ends 132 and 133. Each of the holes has central axis 137, 138, 139, located as a perpendicular to transverse axis of symmetry 128. Besides, in central bulge 131 and in each of ends 132, 133 of the figure plate locating holes 140, 141, 142 are made, the central axes of which are located as a perpendicular to central axes 137, 138, 139 of connecting holes 134, 135, 136.

Figure plate 127 can be made as an isosceles trapezium, in a variant of the fixation junction implement for the purpose to increase fixation rigidity of intact vertebrae and the possibility of controlling position of attachment of fastening ends of the vertebral fixation elements, in view of applied distraction forces (Fig. 3). The trapezium has transverse axis of symmetry 143. Connecting holes 144 and 145 are made on the ends of figure plate 127, and connecting holes 146, 147, 148 are made in the central part of the plate. Each of connecting holes 144, 145, 146, 147, 148 has central axes, perpendicular to the plane of figure plate 127. Locating slots 149, 150, 151, 152 are made at angle 35-45° with respect to the transverse axis of symmetry and they are located in twos, and namely: 149, 150 and 151, 152, on two sides from it and, respectively, between connecting holes, namely: 144, 146 and 145, 147.

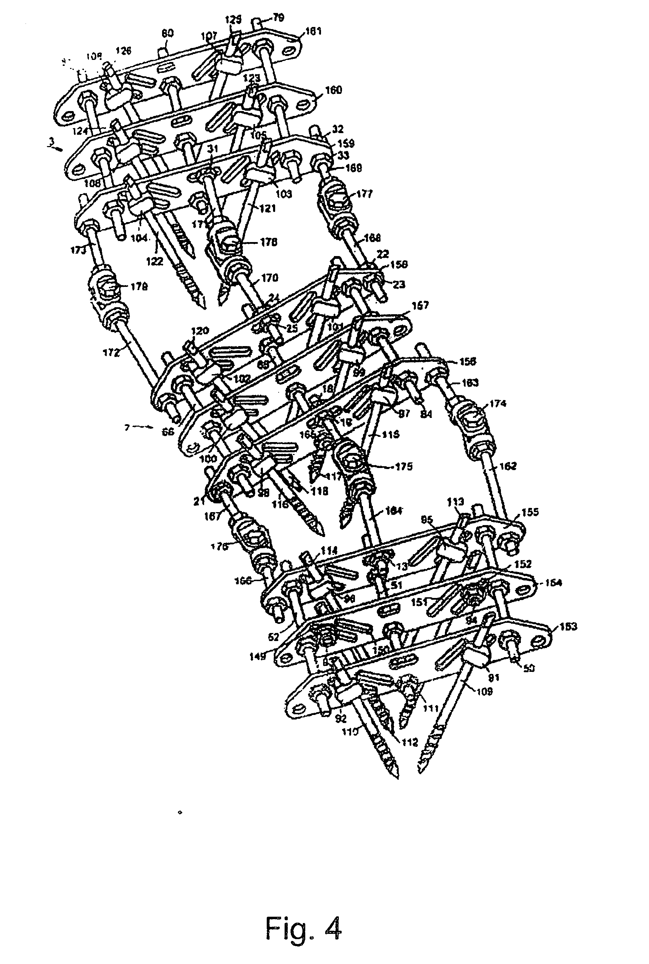

The elements of vertebral fixation 109-126 in one are attached to each of locating slots 129, 130 (Fig. 1) of each fixation junction 46, 47, 48, 49, 62, 63, 76, 77, 78, and the elements of vertebral fixation 109-126 in one as well are attached to each of locating slots 149 or 150 and 151 or 152 (Fig. 4) of each of fixation junctions 153-161. Each of the elements of vertebral fixation is attached by clamps 91-108, respectively.

Moreover, fixation junctions 153-161 (Fig. 4), made as a plate of an isosceles trapezium shape, are connected with each other in threes, and namely: 153, 154, 155, and 156, 157, 158 and 159, 160, 161 by threaded ties 50, 51, 52, and 64, 65, 66 and 79, 80, 81, respectively, set in corresponding connecting holes, thereby forming three support units. One of the support units is intermediate and two others are outer ones. Plates 155, 156 and 158, 159 are interconnected by distraction rods 162, 163, 164, 165, 166, 167, 168, 169, 170, 171, 172, 173 and connecting hinge junctions 174, 175, 176, and 177, 178, 179. Each of connecting hinge junctions 174, 175, 176, 177, 178, 179 is made from two interconnected, with the possibility of rotation, by a connecting element, posts. The axis of rotation of each connecting hinge junction 174, 175, 176, 177, 178, 179 is located in parallel with the plane of each of plates 155, 156 , 158, 159.

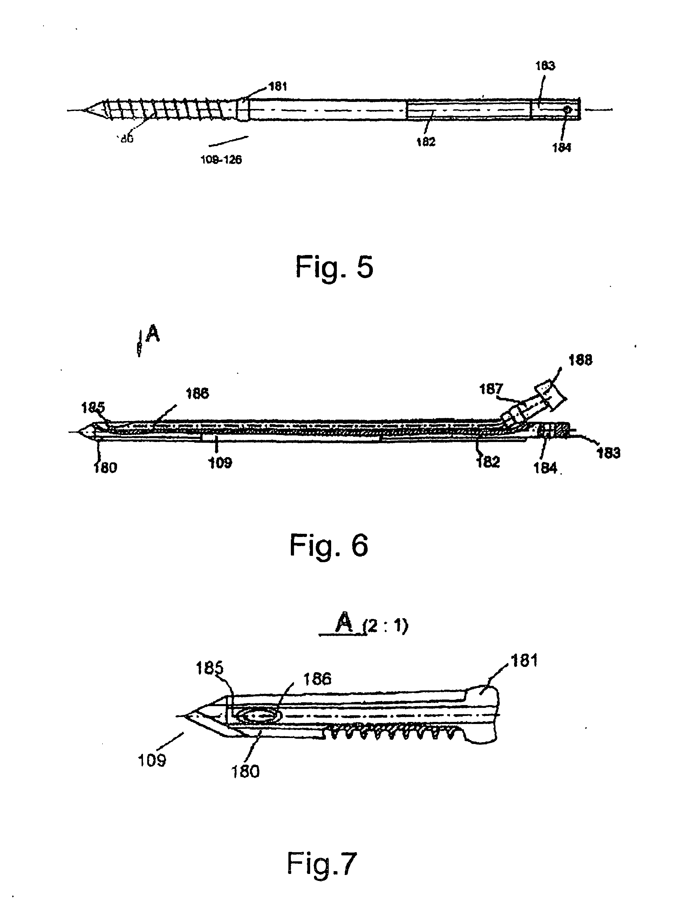

Elements of vertebral fixation 109-126 (Fig. 1, 4), made as half pins, in twos are attached to each of plates 153-161. Each of the half pins has working end 180 (Fig. 5), which is made cone-shaped and provided with screw-like thread, limited stop site 181, and fastening end 182, on which flattening 183 is made, provided with through hole 184. The axis of hole 184 is located as a perpendicular to the longitudinal axis of the half pin.

Each half pin can be provided with longitudinal slot 185, made all over the half pin length (Fig. 6, 7) for the purpose of infusion of medicamentous solutions into bodies of vertebrae during their fixation in the process of treatment. In longitudinal slot 185 syringe needle 186 is fastened, in which cannula 187, provided with plug 188, is set on the side of fastening end 182.

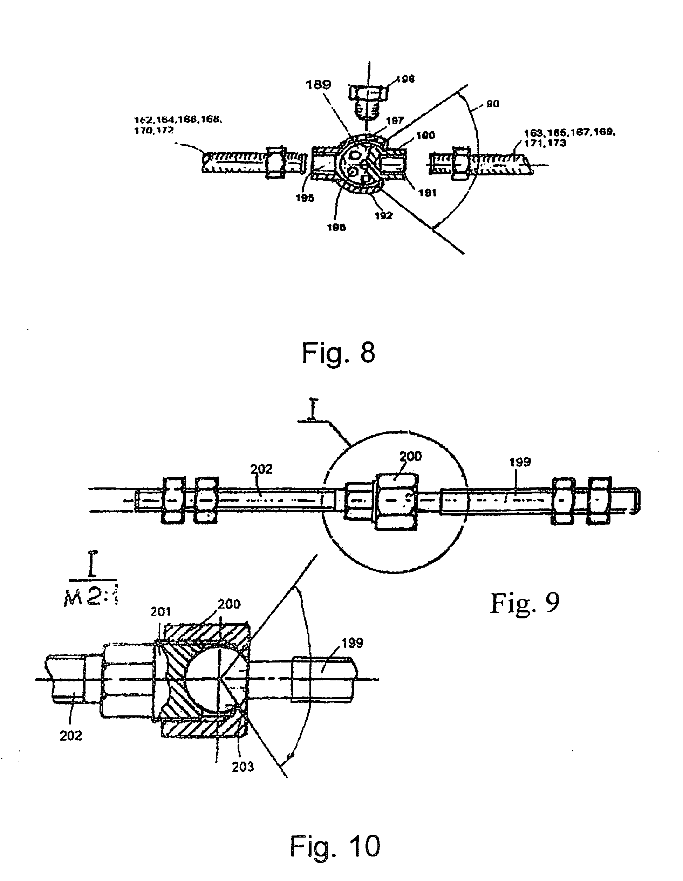

In a variant of the invention implementation each of connecting hinge junctions 174, 175, 176, 177, 178, 179 can be made as ball head 189 (Fig. 8), having cylindrical bulge 190 and hole 161, to obtain complete correction of spinal deformity. Ball head 189 is set in truncated sphere on the side, opposite to cylindrical bulge 190, and hollow sphere 192, the inner surface of which is covered with antifriction coating. Truncated part 193 of sphere 192 comes to 1/3 of its volume, and the angle in projection of truncated part 193 is 90°, that is equal to the maximal angle of spinal scoliotic deformity. Besides, hollow sphere 192 is provided with cylindrical bulge 194 with hole 195. Distraction rods 163, 165, 167, 169, 171, 173, and 162, 164, 166, 168, 170, 172, respectively, are set in holes 191 and 195 of each connecting hinge junction with the possibility of transport. Ball head 189 is provided with depressions 196, and hollow sphere 192 - with hole 197, in which lock 198 is set.

Moreover, each of connecting hinge junctions 174, 175, 176, 177, 178, 179 can be made as distraction rod 199 (Fig. 9, 10), provided on one of the ends with a ball head, set in sleeve 200. The latter is connected with pivot 201, made on one of the ends of another distraction rod 202. In this case projection of bore angle is 90°. The proposed variant of the connecting hinge junctions allows to simplify the process of their production.

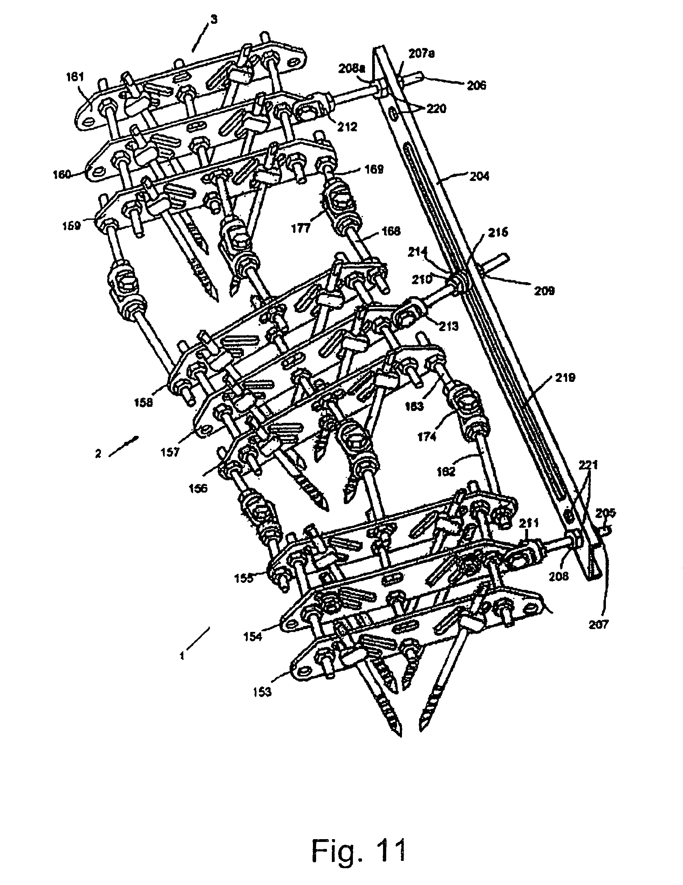

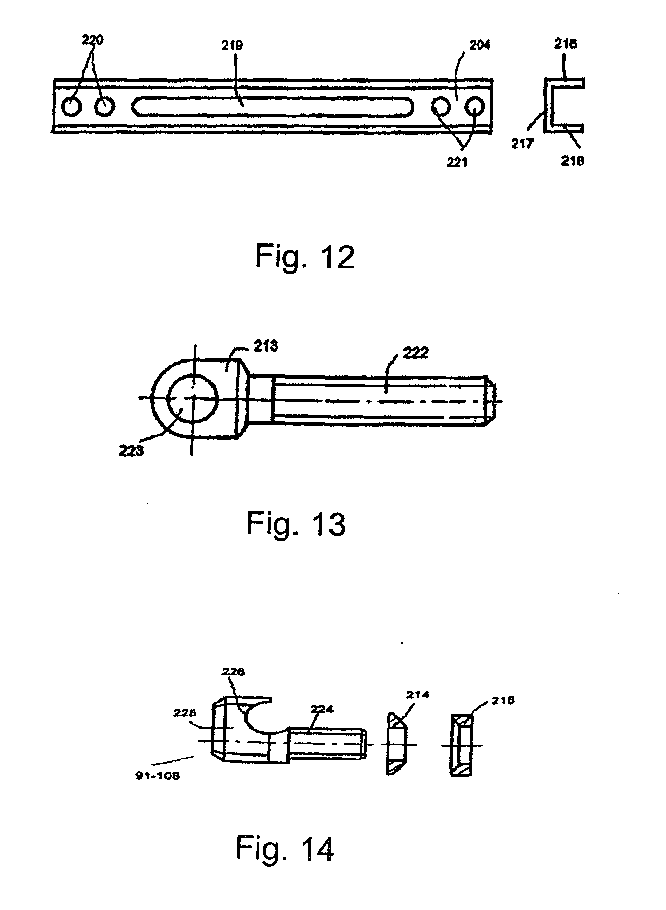

In a variant of the invention implementation the device can be provided with microschweller 204 to achieve additional influence during correction of spinal lateral deformity and to increase the construction rigidity (Fig. 11). Microschweller 204 is connected with posts 211, 212, set on fixation junctions 154, 160, respectively, of outer support units 1 and 3, with the possibility of transport, using threaded rods 205, 206 and two pairs of load-bearing nuts 207, 208, and 209, 210, respectively. Besides, fixation junction 157 of intermediate support unit 2 is connected with microschweller 204, with the possibility of transport, by connecting element 213, of a pair of load-bearing nuts 209, 210 and two pairs of conical 214 and spherical 215 washers, respectively (Fig. 12). The microschweller is made from three planes 216, 217, 218, two of them, namely: 216, 218, are located in parallel and attached to the ends of third plane 217 at angle 90°. A closed slot is longitudinally made on plane 217, and on both sides from it through holes 220, 221, at least in twos are made, the axis of which is a perpendicular to plane 217. In this case connecting element 213 (Fig. 13) is made as a lug with thread shank 222 and through hole 223, and the axis of the lug is a perpendicular to the axis of thread shank 222.

To prevent turning-through of each element of vertebral fixation 109-126, fixed with the help of clamps 91-108, each of the latters (Fig. 1, 4) is made as an angulate post, formed by cylindrical threaded surface 224, connected at right angles with prismatic head 225, on the inner surface of which cylindrical turning-down 226 with cuts is made on the side of the rod. In this case conical 214 and spherical 215 washers are set on cylindrical threaded surface 224 of each of the clamps.

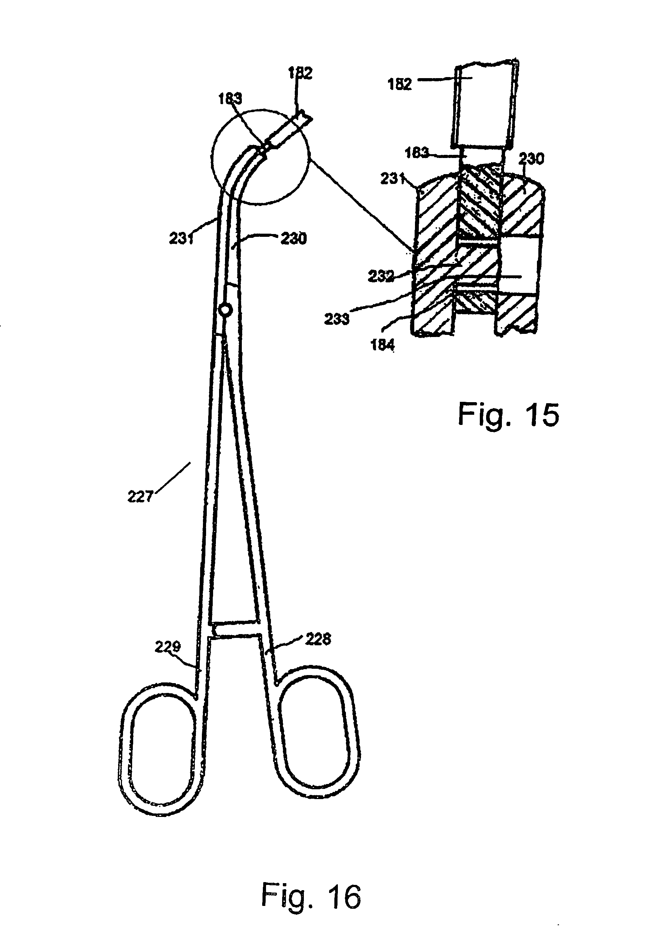

To reduce damage of the soft tissues, surrounded the spine, surgical clamp 227 is used during insertion of vertebral fixation elements 109-126 (Fig. 15). Handles 228, 229 of the surgical clamp has jaws 230, 231. Jaw 231 has boss 232, which is coaxial to hole 233, made in jaw 230. Besides, the diameter of cylindrical bulge 232 shouldn't be above the diameter of hole 184 (Fig. 5), made in flattening 183 of fastening end 182 of the vertebral fixation element.

The device for external transpedicular fixation of the spine, the technique of its use and the surgical clamp for insertion of the vertebral fixation element can be used as follows.

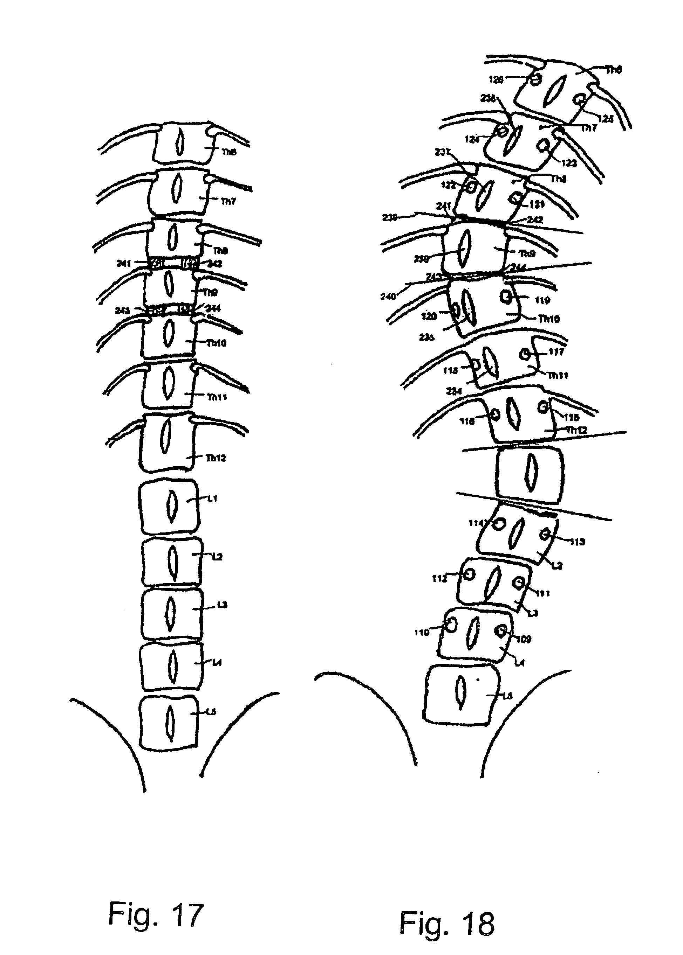

An examination is made before surgical treatment, thereby determining its indications and its scope. In case of scoliosis of III-IV degree after twice-repeated processing of skin it is cut along the line of spinous processes of vertebrae Th₇, Th₈, Th₉, Th₁₀, Th₁₁ under endotracheal anesthesia (Fig. 17). Aponeurosis is cut on both sides from spinous processes 234, 235, 236, 237, 238. The longest muscles of the back are separated with a raspatory, and hemostasis is performed with a hot physiologic salt solution. After that partial resection of vertebral arches (Th₈ and Th₁₀) is made as well as laminectomy of Th₉ vertebra.

Discotomy of discs is performed using a chisel on both sides from spinal cord: 239 cranial vertebrae Th₈-Th₉ and 240 caudal vertebrae Th₉-Th₁₀, which are at the apex of the deformity. Then autografts 241, 242, 243 and 244, respectively, prepared from spinous process 236 and arches of Th₈, Th₉, Th₁₀ vertebrae, are inserted into the cavity of discs 239 and 240.

After that transpedicular fixation of intact vertebrae is performed. Insertion of the vertebral fixation elements in their bodies is made through arch roots; the elements are made as half pins 109-126 (Fig. 14).

Here is an example of insertion of half pin 125 into intact vertebra Th₆

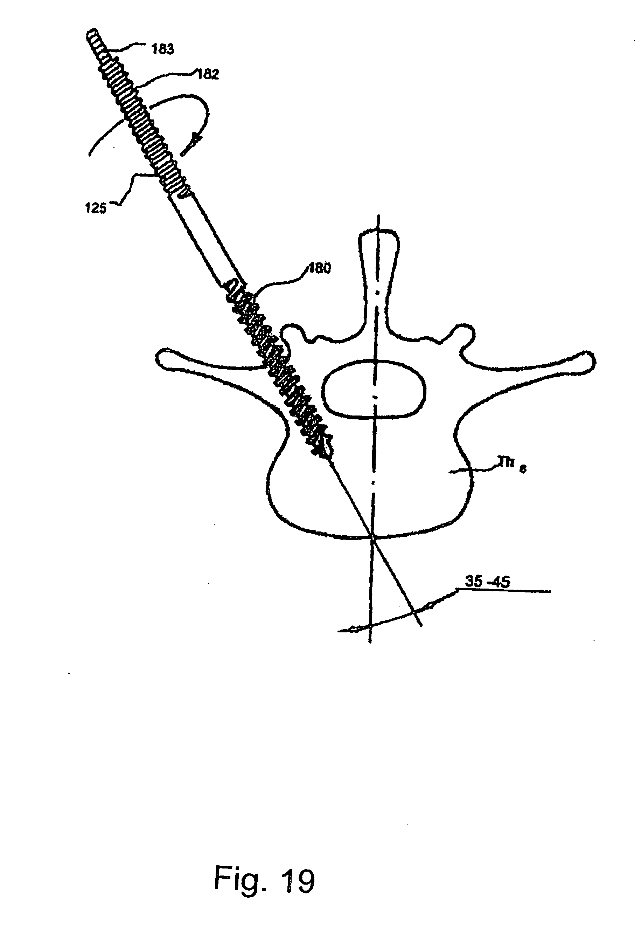

Surgical clamp 227 is used for insertion of half pin 125 through soft tissues (Fig. 15). An additional incision about 1 cm is made in soft tissues in the projection of the pedicle of the arch of Th₆ intact vertebra. A canal is formed from additional incision in soft tissues towards the incision along the line of spinous processes, using jaws 231, 230 of the surgical clamp. After that boss 232 (Fig. 16) of jaws 231 of handle 229 is inserted into hole 184 of flattening 183 of fastening end 182 of half pin 125 (Fig. 1, 4). Flattening 183 is grasped with handles 229, 228 and it is clamped. Fastening end 182 is pulled through the canal, formed in soft tissues. Working end 180 (Fig. 19) of half pin 125 is set in the centre of arch pedicle of intact vertebra Th₆, being oriented at angle 35-45° with respect to sagittal plane 245, and after that it is inserted by twisting into the arch of intact vertebra The.

Half pin 126 (Fig. 1, 4) is inserted similarly, as well as half pins 123, 124, respectively, into intact vertebra Th₇, half pins 121, 122 into intact vertebra Th₈, half pins 119, 120 into intact vertebra Th₁₀, half pins 117, 118 into intact vertebra Th₁₁, half pins 115, 116 into intact vertebra Th₁₂, half pins 113, 114 into intact vertebra L₂, half pins 111, 112 into intact vertebra L₃, half pins 109, 110 into intact vertebra L₄. Layer-by-layer suturing of the incision along the line of the spinous processes of intact vertebrae Th₆, Th₇, The, Th₁₀, Th₁₁, Th₁₂, L₂, L₃, L₄ is performed after respective insertion of half pins 109-126 into these vertebrae. The fastening ends of inserted half pins 109-126 (Fig. 1, 4) are attached, using conical 214 and spherical 215 washers (Fig. 14) and clamps 91-108, each of them is made as angulate posts, which are set in every of longitudinal through slots 129, 130 (Fig. 2) of each fixation junction 78, 77, 76, 63, 62, 47, 46, 48, 49, made as a figure plate. The fastening ends of half pins 125, 126 (Fig. 1) are fixed, respectively, with clamps 107, 108 in fixation junction 78. The fastening ends of half pins 123, 124 are fixed, respectively, with clamps 105, 106 in fixation junction 77. The fastening ends of half pins 121, 122 are fixed, respectively, with clamps 103, 104 in fixation junction 76. The fastening ends of half pins 119, 120 are fixed, respectively, with clamps 101, 102 in fixation junction 63. The fastening ends of half pins 117, 118 are fixed, respectively, with clamps 99, 100 in fixation junction 47. The fastening ends of half pins 113, 114 are fixed, respectively, with clamps 95, 96 in fixation junction 46. The fastening ends of half pins 111, 112 are fixed, respectively, with clamps 93, 94 in fixation junction 48. The fastening ends of half pins 109, 110 are fixed, respectively, with clamps 91, 92 in fixation junction 49.

Made as figure plates fixation junctions 76, 77, 78 (Fig. 1 ), are connected, using threaded ties 79, 80, 81, set in the connecting holes, and are attached with pairs of fastening nuts: 82, 83, 84, 85, 86, 87, 88, 89, 90, thereby forming outer support unit 3. Fixation junctions 47, 62, 63 are connected, using threaded ties 64, 65, 66 and attached with pairs of fastening nuts: 67, 68, 69, 70, 71, 72, 73, 74, 75, thereby forming intermediate support unit 2. And fixation junctions 46, 48, 49 are connected, using threaded ties 50, 51, 52 and attached with matching pairs of fastening nuts: 53, 50, 55, 56, 57, 58, 59, 60, 61, thereby forming the other outer support unit 1. Lugs 34, 35, 36, 40, 41, 42, 43, 44, 45 are connected, using threaded shanks, with the connecting holes of the matching fixation junctions, and namely: 76 - that of outer support unit 3, 63 and 47 - those of intermediate support unit 2, and 46 - that of outer support unit 1. Lugs 43 and 40, 44 and 41, 45 and 42, 37 and 34, 38 and 35, 39 and 36 are connected with the possibility of transport, using distraction threaded rods 7, 8, 9, 6, 5, 4, respectively, and pairs of distraction nuts: 32 and 33, 22 and 23, 30 and 31, 24 and 25, 28 and 29, 26 and 27, 20 and 21, 15 and 14, 18 and 19, 12 and 13, 16 and 17, 10 and 11, respectively.

Graduated transport of support units, outer one 3 (Fig. 1), intermediate one 2, is performed along the concave side of the spine. By untwisting distraction nuts 32 and 23, located on distraction threaded rod 7, lugs 43 and 40 are transported. By twisting distraction pairs of nuts 25 and 24, 30 and 31 lugs 41 and 44, respectively, are transported in the opposite direction releasing the latters for the moment of graduated transport of lugs 43 and 40, which indirectly transfer the applied distraction forces to fixation elements 121, 122, 123, 125, 126 (Fig. 1, 17), inserted in intact vertebrae Th₆, Th₇, Th₈ and to fixation elements 119, 120, 118, 117, 115, 116, inserted in intact vertebrae Th₁₀, Th₁₁, Th₁₂, by their graduated transporting latters in the cranial and caudal directions, respectively. After graduated transport of lugs 43 and 40 (Fig. 1) distraction nuts 33 and 22 are attached to distraction threaded rod 7 and distraction nuts 30 and 31, 24 and 25 - to distraction threaded rod 8. Simultaneously distraction nuts 14 and 20, located on distraction threaded rod 6, should be untwisted. Pairs of distraction nuts 12 and 13, 18 and 19, located on distraction threaded rod 5, are transported in the opposite directions from lugs 35 and 38. After that by indirect twisting of distraction nuts 12 and 21, through lugs 36 and 39, of outer 1 and intermediate 2 support units, fixation elements 110, 112, 114 are transported, and intact vertebrae Th₁₀, Th₁₁, Th₁₂ (Fig. 17), respectively, in the cranial direction, and L₂, L₃, L₄ - in the caudal direction. After graduated transport of lugs 36 and 39 (Fig. 1) distraction nuts 15, 21 are attached to the distraction threaded rod 6, and pairs of distraction nuts 12 and 13, 18 and 19 - to distraction threaded rod 5. Lugs 36, 35 of support unit 1, lugs 39, 38, 42, 41 of support unit 2, and lugs 45, 44 of support unit 3 have the possibility of spatial rotation, thereby taking up a functionally favourable position relative to the distraction threaded rods 6, 5, 7, 8, respectively. Intact vertebrae Th₆, Th₇, Th₈ and Th₁₀, Th₁₁, Th₁₂, L₂, L₃, L₄, located above and below the zone of surgical intervention, are transported graduatedly as it has already been mentioned, and straightening of the spine is achieved (Fig. 18), using the system for external transpedicular fixation. The achieved position of the spine is fixed up to determination of bone or osteofibrous regenerate in the zone of discotomies.

In a variant of the invention implementation each of the fixation junctions is made as plate 127 (Fig. 3) of isosceles trapezium shape with transverse axis of symmetry 143. Plate 127 has connecting holes 144, 146, 148, 147, 145 and through setting slots 149, 150, 151, 152. The latters are located in pairs on both sides from transverse axis of symmetry 143 of the plate and are made at 35...45° angle. During configuration of the device for external transpedicular fixation elements of fixation 109, 110, 112, 113, 114, 115, 116, 117, 118, 119, 120, 121, 122, 123, 124, 125, 126 of intact vertebrae are inserted into their bodies similarly to the technique described above. Support units 1, 2, 3 are configured from the fixation junctions, made as plates 153, 154, 155, 156, 157, 158, 159, 160, 161 (Fig. 4) of isosceles trapezium shape. Fastening ends of two vertebral fixation elements, namely: 109, 110, 111, 112, 113, 114, 115, 116, 117, 118, 119, 120, 121, 122, 123, 124, 125 and 126, respectively, are attached to each of the plates in the through setting slots, using clamps 91 and 92, 93 and 94, 95 and 96, 97 and 98, 99 and 100, 101 and 102, 103 and 104, 105 and 106, 107 and 108, respectively. Outer support unit 1 is formed by connection of plates 153, 154, 155 with threaded ties 50, 51 and 52, which are located in the connecting holes of plates 153, 154 and 155, and it is attached to the latters with pairs of nuts.

Intermediate support unit 2 is formed by connection of plates 156, 157, 158 with threaded ties 64, 65, 66, which are set in the connecting holes of plates 156, 157, 158, and it is attached to the latters with pairs of nuts. Outer support unit 3 is formed by connection of plates 159, 160, 161 with threaded ties 79, 80, 81, which are set in the connecting holes of plates 159, 160, 161, and it is attached to the latters with pairs of nuts.

Outer support unit 1 is connected with intermediate support unit 2 by three pairs of distraction rods 162 and 163, 164 and 165, 166 and 167. Each pair of the latters is connected by the connecting junction, namely: 174, 175, 176. The ends of distraction rods 162 and 163, 164 and 165, 166 and 167 of each pair are set in the connecting holes of respective plates 155 and 156 with the possibility of transport. In this case the axis of each connecting hinge junction is set in parallel with the sagittal plane.

Intermediate support unit 2 is connected with outer support unit 3 by three pairs of distraction rods 168 and 169, 170 and 171, 172 and 173. Each pair of the latters is connected by the connecting junction, namely: 177, 178, 179. The ends of distraction rods 168 and 169, 170 and 171, 172 and 173 of each pair are set in the connecting holes of respective plates 158 and 159 with the possibility of transport. In this case the axis of each connecting hinge junction is set in parallel with the sagittal plane. Distraction nuts 13, 12 (not shown) and 19, 18, respectively, on distraction rods 164 and 165, distraction nuts 25, 24 and 31, 30 (not shown) on distraction rods 170 and 171 are untwisted in the opposite directions from plates 155, 156 and 158, 159, respectively, to implement graduated transport of intact vertebrae. Besides, distraction nuts 52, 20 (not shown) on distraction rods 164 and 167 and distraction nuts 23, 32 on distraction rods 168 and 169 are untwisted. After that distraction nuts 21 and 15 (not shown) should be graduatedly transported along distraction rods 166 and 167, and distraction nuts 22 and 33 - along distraction rods 168, 169. Distraction nuts 13, 12 (not shown), 19, 18, 14, 15 (not shown), 21, 20 (not shown),22, 23, 33, 32, 25, 24, 31, 30 (not shown) are fastened. Graduated transport is performed periodically till the spine normocorrection planned is achieved.

To obtain an additional effect on the spine during correction of its marked deformity and to increase the design rigidity fixation junctions 154, 160, respectively (Fig. 11), of the outer support units and fixation junction 157 of intermediate support unit 2 are connected with microschweller 204 (Fig. 11, 12). The latter is made from plane 217, which is connected with planes 216 and 218 at right angles. Through holes 220, 221, and closed longitudinal slot 219, are made in plane 217.

Threaded end 222 (Fig. 13) of lug 213, attached with the help of load-bearing nuts 209, 210, is set in closed longitudinal slot 219 (Fig. 11), using conical 214 and spherical 215 washers. Lug 213 is connected with the connecting hole of fixation junction 157 (Fig. 11), using through hole 223 and a bolt connection. One of the through holes on both sides from closed longitudinal slot 219 is connected with threaded rods 205 and 206, respectively, using two pairs of load-bearing nuts 207, 208 and 207a and 208a. The threaded rods are connected with posts 211 and 212, respectively, which are attached to fixation junctions 154 and 160, respectively, with the help of bolt connections. To obtain additional traction, pairs of load-bearing nuts 207, 208 and 207a, 208a on threaded rods 205 and 206 are untwisted in different directions from microschweller 204, and load-bearing nut 209 on the threaded shank of lug 213 is untwisted as well. Then graduated twisting of load-bearing nut 210 is performed, and required transport of microschweller 204 in the transverse plane is made with the help of this nut. After transportation load-bearing nuts 207, 208, 207a, 208a, 210 are fixed. The graduated transport in the transverse plane should be repeated if necessary, using the described technique.

To infuse medicamentous agents in bodies of vertebrae during their fixation in the process of treatment elements of vertebral fixation 109, 110, 111, 112, 113, 114, 115, 116, 117, 118, 119, 120, 121, 122, 123, 124, 125, 126 are used, each of them is made as half pin 109 (Fig. 6, 7). Longitudinal slot 185 is made all over the length of the latter, where syringe needle 186 is fixed. The end of syringe needle 186 is located near the point of working end 180 of half pin 109, and it is provided with cannula 187 and plug 188 on the side of fastening end 182. Insertion and fastening of half pin 109, provided with syringe needle 186, in the fixation junction is performed in the similar way, using the technique, described above. After that, if necessary, for the purpose of prevention or treatment of inflammations plug 188 should be removed from cannula 187, and a medicamentous agent is infused by stream or by drops through syringe needle 186, fixed in half pin 109, which is inserted in the body of an intact vertebra.

To achieve complete correction of the spine deformities both for trauma and for scoliosis of III-IV degree multiaxial connecting hinge junction can be used in the configuration of the device for external transpedicular fixation for connection of support units 1, 2, 3; ball head 189 in this junction (Fig. 8), which has cylindrical bulge 190, is set in hollow sphere 192. Cylindrical bulge 190 in this case is located on the side of truncation, made in the hollow sphere and equal to its 1/3, and a projection angle is equal to 90°, being the greatest angle of scoliotic deformity of the spine. The hollow sphere is provided with cylindrical bulge 194 on the outer surface. Holes 191 and 195, respectively, are made in cylindrical bulges 190 and 194. In each of the holes distraction threaded rods 163, 165, 167, 169, 171, 173 and 162, 164, 166, 168, 170, 172 are set , respectively. For fixation of spatial position depressions 196 are made on ball head 189, and hole 197 is made in hollow sphere 192, lock 198 is set in this hole. The inner surface of hollow sphere 192 is covered with antifriction coating. As for the mentioned connecting hinge junctions, from 2 to 6 of them can be used in the configuration of the device for external transpedicular fixation.

Moreover, each of the connecting hinge junctions can be made as half pin 199 (Fig. 9, 10), and a ball head is made on one of its ends. The head is located in sleeve 200 with a bore, angle 203 of which is 90°, conforming to the greatest angle of scoliotic deformity. The ball head of distraction rod 199 is held in sleeve 200 with the help of pivot 201, which is made on the end of distraction rod 202. In this case pivot 201 is connected with sleeve 200 by thread. 2 - 6 connecting hinge junctions of the design described can be used in the configuration of the device for external transpedicular fixation.

### Industrial Usability

The patented invention is not limited by the variants of its implementation and use, described above, and includes other possible modifications within the applied claims.

The device for external transpedicular fixation of the spine, proposed for patenting, in view of minimal nomenclature of its parts included, allows to assemble configurations of the devices, intended for treatment of the spine diseases of different etiology: fresh and old dislocations, injuries, spondylolistheses, scolioses, kyphoses.

The proposed device gives the possibility of simultaneous fixation of both anterior and posterior structures of the spine, thereby causing rigid fixation of vertebrae, high control ability of intact vertebrae position, provides for rigidity of *device - spine* system.

Besides, provision of secure contact with vertebrae allows to apply strictly graduated forces of distraction to the spine in any direction and at any stage of treatment.

## Claims

1. A device for external transpedicular fixation of a spinal column comprising
- fixation units (46-49; 62, 63; 76-78) provided with holes and connected with each other by threaded ties (50-52; 64-66; 79-81), thereby forming support units (1-3) connected to one another by means of distraction threaded rods (4-6; 7-9) with a possibility of movement relative to each other,
- wherein on each fixation unit (46-49; 62, 63; 76-78) are mounted two vertebral fixation elements (109 - 126), one fixing end of which is fastened to the fixation unit (46-49; 62, 63; 76-78) by clamps (91-108),
- wherein each of the vertebral fixation elements (109 - 126) is formed as a screw-pin having
■ a working end (180) provided with a screw-like thread with a cutting edge limited by a stop site (181), and
■ a fastening end (182),
- wherein each fixation unit (46,48,49; 47,62,63; 76,77,78) is
■ made in the form of a figure plate (127, 127a) with a transverse symmetry axis (128, 143) and
■ provided with through holes (134, 135, 136, 140, 141, 142, 144, 145, 146, 147) and with symmetrical longitudinal through slots (129,130,149,150,151,152),
**characterized in that** the device comprises
- nine fixation units (46-49; 62, 63; 76-78), not more, which are assembled to form three support units (1 - 3);
- wherein each support unit (1-3) comprises three fixation units (46,48,49; 47,62,63; 76,77,78) fixed by threaded ties (50-52; 64-66; 79-81);
- wherein the support units (1-3) are connected to the distraction threaded rods (4-6; 7-9) by lugs (34,35,36; 37,38,39; 40,41,42; 43,44,45) with threaded shank ends, providing the possibility of spatial movement; and
- wherein each fastening end (182) of the vertebral fixation elements (109 - 126) has a flattening (183) provided with a through hole (184) which axis is perpendicular to the longitudinal axis of the screw-pin.

2. Device according to claim 1, **characterized in that** each of the fixation unit (46-49; 62, 63; 76-78) is provided
- with a central bulge (131) and ends (132, 133), each of them being made in the form of a prism, having attaching holes (134, 135, 136) and locating holes (140, 141, 142), and
- wherein the symmetrical longitudinal through slots (129,130) are located between the central bulge (131) and the ends (132, 133) of the fixation unit (46-49; 62, 63; 76-78).

3. Device according to claim 1, **characterized in that**
- the figure plate (127a) of each of the fixation units (46, 47, 48, 49, 62, 63, 76, 77, 78) is carried out as an isosceles trapezium (127a);
- the figure plate (127a) is provided with connecting through holes (144, 145, 146, 147) being symmetrical with regard to a transverse axis (143) of the figure plate (127a), wherein the axes of the connecting through holes (144, 145, 146, 147) are perpendicular to the transverse axis (143) of the figure plate (127a);
- wherein the connecting holes (144, 145; 146, 147) are provided on the ends and in the middle of the figure plate (127a);
- wherein each of the figure plates (127a) is provided with two pairs of mounting through slots (149,150; 151, 152), made at an angle of 35° to 40° on both sides from the transverse axis (143) of the figure plate (127a);
- the support units (1,2,3) are connected with the possibility of spatial movement by means of connecting hinge junctions (174, 175, 176; 177, 178, 179), each of which is assembled from interconnected posts, and each of the latter is provided with distraction rods (162, 163; 164, 165; 166, 167; 168, 169; 170, 171; 172, 173).

4. Device according to one of the preceding claims, **characterized in that**
- the screw-pin is provided with a longitudinal slot (185) over its length for fixing a syringe needle (186) therein;
- wherein a cannula (187) provided with a plug (188) is insertable in the syringe needle (186) on the side of the fastening end (182) of the vertebral fixation element (109 - 126).

5. Device according to claim 3, **characterized in that**
- each of said connecting hinge junctions (174, 175, 176, 177, 178, 179) is formed as a ball head (189) located in a hollow sphere (192);
- the ball head (189) and the hollow sphere (192) are provided with cylindrical bulges (190, 194) having holes (191, 195);
- wherein a truncated part (193) is provided on the opposite side of the hollow sphere (192);
- wherein an inner surface of the hollow sphere (192) is covered with an antifriction coating;
- wherein the distraction rods (162 - 173) are arranged in the holes (191, 195) of the cylindrical bulges (190, 194),
- wherein the truncated part (193) of the hollow sphere (192) composes 1/3 of its volume, and the projection angle of the truncated part (193) is 90°;
- wherein the ball head (189) is provided with recesses (196) and the hollow sphere (192) is provided with a hole (197), in which a lock (198) is arranged;
- wherein the axis of the hole (197) is perpendicular to the axis of the holes (191, 195) of the cylindrical bulges (190, 194).

6. Device according to claim 3 or 5, **characterized in that**
- each of said connecting hinge junctions (174 - 179) is carried out as a first distraction rod (199), provided with a ball head on one of its ends;
- wherein the ball head of the first distraction rod (199) is connectable by a sleeve (200) to a pivot (201), made on one of the ends of a second distraction rod (202).

7. Device according to claim 3, **characterized by**
- a microschweller (204), provided with a closed longitudinal slot (219) on one of its planes, wherein on each side of the longitudinal slot (219) are arranged at least two settling through holes (220, 221);
- wherein the microschweller (204) is connected to the support units (1, 3) by holders (211, 212) and threaded rods (205, 206), and
- wherein the microschweller (204) is connected with the intermediate support unit (2) with the possibility of movement using a lug (213) with a threaded shank.

8. Device according to one of the claims 1, 2, 3 or 6, **characterized in that**
- each of the clamps (91 - 108) is carried out as an angle rod formed by cylindrical threaded surface (224) connected with a prismatic head (225) at an angle;
- wherein the axis of the prismatic head (225) is positioned in eccentricity with regard to the axis of the cylindrical threaded surface (224);
- wherein a cylindrical cavity (226) is provided on the side of the rod;
- wherein conical and spherical washers (214, 215) are provided on the cylindrical threaded surface (224).

## Patentansprüche

1. Vorrichtung zur externen transpedikularen Fixation einer Wirbelsäule mit
- Fixationseinheiten (46-49; 62, 63; 76-78), die mit Öffnungen versehen sind und miteinander durch mit Gewinde versehenen Zugelementen (50-52; 64-66; 79-81) verbunden sind, wodurch sie Stützeinheiten (1-3) bilden, die miteinander mittels mit Gewinde versehener Distraktionsstangen (4-6; 7-9) mit einer Möglichkeit für eine relative Bewegung zueinander verbunden sind,
- wobei an jeder Fixationseinheit (46-49; 62, 63; 76-78) zwei vertebrale Fixationselemente (109-126) angebracht sind, deren eines Fixierende an der Fixationseinheit (46-49; 62, 63; 76-78) durch Klemmen (91-108) befestigt ist,
- wobei jedes der vertebralen Fixationselemente (109-126) als Schraubenstift ausgebildet ist, der
- ein Arbeitsende (180), das mit einem schraubenähnlichen Gewinde mit einer durch einen Anschlagort (181) begrenzten Schneidkante versehen ist, und
- ein Befestigungsende (182) aufweist,
wobei jede Fixationseinheit (46, 48, 49; 47, 62, 63; 76, 77,78)
- als Formplatte (127, 127a) mit einer symmetrischen Querachse (128, 143) ausgebildet ist und
- mit Durchgangsöffnungen (134, 135, 136, 140, 141, 142, 144, 145, 146, 147) und mit symmetrischen länglichen Durchgangsschlitzen (129, 130, 149, 150, 151, 152) versehen ist,
**dadurch gekennzeichnet, dass** die Vorrichtung
- neun Fixationseinheiten (46-49; 62, 63; 76-78), nicht mehr, umfasst, die zur Bildung von drei Stützeinheiten (1-3) zusammengebaut sind;
- wobei jede Stützeinheit (1-3) drei Fixationseinheiten (46, 48, 49; 47, 62, 63; 76, 77, 78) umfasst, die durch mit Gewinde versehene Zugelemente (50-52; 64-66; 79-81) befestigt sind;
- wobei die Stützeinheiten mit den mit Gewinde versehenen Distraktionsstangen (4-6; 7-9) durch Ansätze (34, 35, 36; 37, 38, 39; 40, 41, 42; 43, 44, 45) mit mit Gewinde versehenen Schaftenden verbunden sind,
wobei die Möglichkeit einer räumlichen Bewegung geschaffen wird, und
- wobei jedes Befestigungsende (182) der vertebralen Fixationselemente (109-126) eine Abflachung (183) aufweist, die mit einer Durchgangsöffnung (184) versehen ist, deren Achse senkrecht zu der Längsachse des Schraubenstiftes verläuft.

2. Vorrichtung nach Anspruch 1, **dadurch gekennzeichnet, dass** jede der Fixationseinheiten (46-49; 62, 63; 76-78)
- mit einer zentralen Ausbauchung (131) und Enden (132, 133) versehen ist, die jeweils in Form eines Prismas mit Befestigungsöffnungen (134, 135, 136) und Einstellöffnungen (140, 141, 142) ausgebildet sind, und
- wobei die symmetrischen länglichen Durchgangsschlitze (129, 130) zwischen der zentralen Ausbauchung (131) und den Enden (132, 133) der Fixationseinheiten (46-49; 62,63; 76-78) angeordnet sind.

3. Vorrichtung nach Anspruch 1, **dadurch gekennzeichnet, dass**
- die Formplatte (127a) jeder der Fixationseinheiten (46, 47, 48, 49, 62, 63, 76, 77, 78) als gleichschenkliges Trapez (127a) ausgeführt ist;
- die Formplatte (127a) mit Verbindungsdurchgangsöffnungen (144, 145, 146, 147) versehen ist, die bezüglich einer Querachse (143) der Formplatte (127a) symmetrisch sind, wobei die Achsen der Verbindungsdurchgangsöffnungen (144, 145, 146, 147) senkrecht zu der Querachse (143) der Formplatte (127a) sind,
- wobei die Verbindungsöffnungen (144, 145; 146, 147) an den Enden und in der Mitte der Formplatte (127a) vorgesehen sind,
- wobei jede der Formplatten (127a) mit zwei Paaren von Montagedurchgangsschlitzen (149, 150; 151, 152) versehen ist, die in einem Winkel von 35° bis 40° an beiden Seiten von der Querachse (143) der Formplatte (127a) ausgebildet sind,
- die Stützeinheiten (1,2,3) mit der Möglichkeit einer räumlichen Bewegung mittels Verbindungsgelenkverbindungen (174, 175, 176; 177, 178, 179) verbunden sind, die jeweils aus miteinander verbundenen Ständern zusammengebaut sind, und jeder der letztgenannten mit Distraktionsstangen (162, 163; 164, 165; 166, 167; 168, 169; 170, 171; 172, 173) versehen ist.

4. Vorrichtung nach einem der vorhergehenden Ansprüche, **dadurch gekennzeichnet, dass**
- der Schraubenstift über seine Länge mit einem Längsschlitz (185) zur Fixierung einer Spritzennadel (186) darin versehen ist,
- wobei eine mit einem Stopfen (188) versehene Kanüle (187) in die Spritzennadel (186) auf der Seite des Befestigungsendes (182) des vertebralen Fixationselements (109-126) einsetzbar ist.

5. Vorrichtung nach Anspruch 3, **dadurch gekennzeichnet, dass**
- jede der Verbindungsgelenkverbindungen (174, 175, 176, 177, 178, 179) als Kugelkopf (189) ausgebildet ist, der in einer Hohlkugel (192) angeordnet ist,
- der Kugelkopf (189) und die Hohlkugel (192) mit zylindrischen Ausbuchtungen (190, 194) mit Öffnungen (191, 195) versehen sind,
- ein kegelstumpfförmiger Teil (193) an der entgegengesetzten Seite der Hohlkugel (192) vorgesehen ist,
- wobei eine Innenfläche der Hohlkugel (192) mit einer Antireibungsbeschichtung bedeckt ist,
- wobei die Distraktionsstangen (162-173) in den Öffnungen (191, 195) der zylindrischen Ausbuchtungen (190, 194) angeordnet sind,
- wobei der kegelstumpfförmige Teil (193) der Hohlkugel (192) ein Drittel seines Volumens ausmacht und der Projektionswinkel des kegelstumpfförmigen Teils (193) 90° ist,
- wobei der Kugelkopf (189) mit Ausnehmungen (196) und die Hohlkugel (192) mit einer Öffnung (197) versehen ist, in der eine Sperre (198) angeordnet ist,
- wobei die Achse der Öffnung (197) senkrecht zu der Achse der Öffnungen (191, 195) der zylindrischen Ausbauchungen (190, 194) ist.

6. Vorrichtung nach Anspruch 3 oder 5, **dadurch gekennzeichnet, dass**
- jede der Verbindungsgelenkverbindungen (174-179) als eine erste Distraktionsstange (199) ausgebildet ist, die an einem ihrer Enden mit einem Kugelkopf versehen ist,
- wobei der Kugelkopf der ersten Distraktionsstange (199) durch eine Hülse (200) mit einem Drehzapfen verbindbar ist, der an einem der Enden einer zweiten Distraktionsstange (202) ausgebildet ist.

7. Vorrichtung nach Anspruch 3, **gekennzeichnet durch**
- einen Mikroschweller (204), der mit einem geschlossenen Längsschlitz (219) auf einer seiner Ebenen versehen ist, wobei an einer Seite des Längsschlitzes (219) wenigstens zwei Setzdurchgangsöffnungen (220, 221) angeordnet sind,
- wobei der Mikroschweller (204) mit den Stützeinheiten (1, 3) **durch** Halter (211, 212) und Gewindestangen (205, 206) verbunden ist, und
- der Mikroschweller (204) mit der zwischenliegenden Stützeinheit (2) bewegbar verbunden ist, wobei ein Ansatz (213) mit einem Gewindeschaft verwendet wird.

8. Vorrichtung nach einem der Ansprüche 1, 2, 3 oder 6, **dadurch gekennzeichnet, dass**
- jede der Klemmen (91-108) als Winkelstange ausgeführt ist, die von einer zylindrischen Gewindefläche (224) gebildet wird, die mit einem Prismakopf (225) in einem Winkel verbunden ist,
- wobei die Achse des Prismakopfs (225) exzentrisch bezüglich der Achse der zylindrischen Gewindefläche (224) angeordnet ist,
- wobei ein zylindrischer Hohlraum (226) auf einer Seite der Stange vorgesehen ist,
- wobei konische und sphärische Scheiben (214, 215) auf der zylindrischen Gewindefläche (224) vorgesehen sind.

## Revendications

1. Dispositif de fixation transpédiculaire externe d'une colonne vertébrale, comprenant
- des unités de fixation (46 - 49; 62, 63; 76 - 78) munies de trous et raccordées les unes aux autres par des attaches filetées (50 - 52; 64 - 66; 79 - 81), formant ainsi des unités de support (1 - 3) raccordées les unes aux autres au moyen de tiges filetées de distraction (4 - 6; 7 - 9) avec une possibilité de mouvement les unes par rapport aux autres,
- dans lequel, sur chaque unité de fixation (46 - 49; 62, 63; 76 - 78), sont montés deux éléments de fixation vertébraux (109 - 126) dont une extrémité de fixation est fixée à l'unité de fixation (46 - 49; 62, 63; 76 - 78) par des colliers de serrage (91 - 108),
- dans lequel chacun des éléments de fixation vertébraux (109 - 126) est formé comme une broche à vis comportant
■ une extrémité active (180) munie d'un filetage à vis avec une arête tranchante limitée par un site d'arrêt (181), et
■ une extrémité de fixation (182),
dans lequel chaque unité de fixation (46, 48, 49; 47, 62, 63; 76, 77, 78) est
■ réalisée sous la forme d'une plaque profilée (127, 127a) ayant un axe de symétrie transversal (128, 143) et
■ munie de trous traversants (134, 135, 136, 140, 141, 142, 144, 145, 146, 147) et de fentes traversantes longitudinales symétriques (129, 130, 149, 150, 151, 152),
**caractérisé en ce que** le dispositif comprend
- neuf unités de fixation (46 - 49; 62, 63; 76 - 78), pas plus, qui sont assemblées pour former trois unités de support (1 - 3);
- dans lequel chaque unité de support (1 - 3) comprend trois unités de fixation (46, 48, 49; 47, 62, 63; 76, 77, 78) fixées par des attaches filetées (50 - 52; 64 - 66; 79 - 81);
- dans lequel les unités de support (1 - 3) sont raccordées aux tiges filetées de distraction (4 - 6; 7 - 9) par des tenons (34, 35, 36; 37, 38, 39; 40, 41, 42; 43, 44, 45) ayant des extrémités de tige filetées, offrant la possibilité d'un mouvement spatial; et
- dans lequel chaque extrémité de fixation (182) des éléments de fixation vertébraux (109 - 126) comporte un aplatissement (183) muni d'un trou traversant (184) dont l'axe est perpendiculaire à l'axe longitudinal de la broche à vis.

2. Dispositif selon la revendication 1, **caractérisé en ce que** chacune des unités de fixation (46 - 49; 62, 63; 76 - 78) est munie
- d'un renflement central (131) et d'extrémités (132, 133), chacun d'eux étant réalisé sous la forme d'un prisme, comportant des trous d'attache (134, 135, 136) et des trous d'indexage (140, 141, 142), et
- dans lequel les fentes traversantes longitudinales symétriques (129, 130) sont situées entre le renflement central (131) et les extrémités (132, 133) de l'unité de fixation (46 - 49; 62, 63; 76 - 78).

3. Dispositif selon la revendication 1, **caractérisé en ce que**
- la plaque profilée (127a) de chacune des unités de fixation (46, 47, 48, 49, 62, 63, 76, 77, 78) est réalisée comme un trapèze isocèle (127a);
- la plaque profilée (127a) est munie de trous traversants de raccordement (144, 145, 146, 147) symétriques par rapport à un axe transversal (143) de la plaque profilée (127a), les axes des trous traversants de raccordement (144, 145, 146, 147) étant perpendiculaires à l'axe transversal (143) de la plaque profilée (127a);
- dans lequel les trous de raccordement (144, 145; 146, 147) sont disposés sur les extrémités et au milieu de la plaque profilée (127a);
- dans lequel chacune des plaques profilées (127a) est munie de deux paires de fentes traversantes de montage (149, 150; 151, 152) réalisées à un angle de 35° à 40° des deux côtés de l'axe transversal (143) de la plaque profilée (127a);
- les unités de support (1, 2, 3) sont raccordées avec la possibilité d'un mouvement spatial au moyen de jonctions articulées de raccordement (174, 175, 176; 177, 178, 179), dont chacune est assemblée à partir de montants interconnectés, et chacune de ces dernières est munie de tiges de distraction (162, 163; 164, 165; 166, 167; 168, 169; 170, 171; 172, 173).

4. Dispositif selon l'une des revendications précédentes, **caractérisé en ce que**
- la broche à vis est munie d'une fente longitudinale (185) sur sa longueur pour fixer là-dedans une aiguille à injection (186);
- dans lequel une canule (187) munie d'un bouchon (188) est insérable dans l'aiguille à injection (186) du côté de l'extrémité de fixation (182) de l'élément de fixation vertébral (109 - 126).

5. Dispositif selon la revendication 3, **caractérisé en ce que**
- chacune desdites jonctions articulées de raccordement (174, 175, 176, 177, 178, 179) est formée comme une tête à rotule (189) située dans une sphère creuse (192);
- la tête à rotule (189) et la sphère creuse (192) sont munies de renflements cylindriques (190, 194) comportant des trous (191, 195);
- dans lequel une partie tronquée (193) est disposée du côté opposé de la sphère creuse (192);
- dans lequel une surface intérieure de la sphère creuse (192) est revêtue d'un revêtement antifriction;
- dans lequel les tiges de distraction (162 - 173) sont disposées dans les trous (191, 195) des renflements cylindriques (190, 194),
- dans lequel la partie tronquée (193) de la sphère creuse (192) compose 1/3 de son volume, et l'angle de projection de la partie tronquée (193) est de 90°;
- dans lequel la tête à rotule (189) est munie d'évidements (196) et la sphère creuse (192) est munie d'un trou (197), dans lequel un verrou (198) est disposé;
- dans lequel l'axe du trou (197) est perpendiculaire à l'axe des trous (191, 195) des renflements cylindriques (190, 194).

6. Dispositif selon la revendication 3 ou 5, **caractérisé en ce que**
- chacune desdites jonctions articulées de raccordement (174 - 179) est réalisée comme une première tige de distraction (199), munie d'une tête à rotule sur l'une de ses extrémités;
- dans lequel la tête à rotule de la première tige de distraction (199) peut être raccordée par un manchon (200) à un pivot (201), réalisé sur l'une des extrémités d'une seconde tige de distraction (202).

7. Dispositif selon la revendication 3, **caractérisé par**
- un microschweller (204), muni d'une fente longitudinale fermée (219) sur l'un de ses plans, dans lequel au moins deux trous traversants de réglage (220, 221) sont disposés de chaque côté de la fente longitudinale (219);
- dans lequel le microschweller (204) est raccordé aux unités de support (1, 3) par des supports (211, 212) et des tiges filetées (205, 206), et
- dans lequel le microschweller (204) est raccordé à l'unité de support intermédiaire (2) avec la possibilité d'un mouvement utilisant un tenon (213) ayant une tige filetée.

8. Dispositif selon l'une des revendications 1, 2, 3 ou 6, **caractérisé en ce que**
- chacun des colliers de serrage (91 - 108) est réalisé sous la forme d'une tige angulaire formée par une surface filetée cylindrique (224) raccordée à une tête prismatique (225) à un angle;
- dans lequel l'axe de la tête prismatique (225) est positionné de manière excentrique par rapport à l'axe de la surface filetée cylindrique (224);
- dans lequel une cavité cylindrique (226) est disposée sur le côté de la tige;
- dans lequel des rondelles conique et sphérique (214, 215) sont disposées sur la surface filetée cylindrique (224).
